# EUROPEAN PATENT APPLICATION

(11) **EP 2 335 561 A1**
(43) Date of publication of application: **22.06.2011**
(21) Application number: 09812672.5
(22) Date of filing: 11.09.2009
(51) Int. Cl.: A61B 1/307, A61B 1/00

(54) **URETERAL ENDOSCOPE OF ADJUSTABLE SOFTNESS AND RIGIDITY**

(30) Priority: 12.09.2008 CN 200820153056 U; 19.09.2008 CN 200820153290 U; 19.09.2008 CN 200820153291 U
(71) Applicant: Shanghai First People's Hospital, Shanghai 200080 (CN)
(72) Inventor: Xia, Shujie, Hong Kou District Shanghai 200080 (CN); Zhao, Wej, Hong Kou District Shanghai 200080 (CN)
(74) Representative: Denjean, Eric
(86) International application number: PCT/CN2009/073898
(87) International publication number: WO 2010/028606

(57) **Abstract**

The utility model relates to medical apparatus, and in particular to a rigid/flexible adjustable ureteroscope which includes a body, an operation shank and a rigid/flexible adjustment support rod. A rigidity adjustment key controls the rigid/flexible adjustment support rod to be movable freely in the body so that the rigid/flexible adjustment support rod channel. Movement of the rigid/flexible adjustment support rod makes a part of the endoscope becomes rigid so as to have adequate rigidity to overcome resistance, avoiding the needs of hard sheath while entering a uterer. A front part of the endoscope may become from rigid to flexible by movement of the support rod, and the flexible front part may vary in length to accomplish the operation flexibly. The support rod is able to be movable or be drawn out from a support rod channel to serve the support rod channel as a work channel. By adjustment of sizes, a rigid/flexible adjustment fibronephorscope is provided, and alternatively, a rigid/flexible adjustment fiber urethra cystoscope is provided. Compared with the prior art, the utility model overcomes defects of various urethra cystoscopes, raises surgical efficacy and economic merits, avoids hurt to tissues, and reduces apparatus acquisition cost.

## Description

### Field of the Invention

The utility model relates to the field of medical surgery equipment, and relates to an endoscopy for treating ureteral disease, and in particular to rigid/flexible adjustable ureteroscope, which may serve as rigid/flexible adjustable fibronephorscope, or an adjustable fiber urethra cystoscope by means of adjusting sizes thereof.

### Background of the Invention

Ureteroscopes are required in medical clinical diagnoses and treatment of ureteral disease. A conventional ureteroscope is classified as two types, rigid ureteroscope and flexible ureteroscope. The rigid ureteroscope is widely employed, but may hurt patients more seriously and producing complications due to material. Moreover, the rigid ureteroscope is an optical scopy, which typically has visual angles of 0 degree and five degrees, resulting in poor image quality of optical system. Continuous bladder wash is needed to guarantee distinct surgery visual field. The fiber urethra cystoscope, i.e. a flexible cystoscope, cannot enter a catheter unless assistance of hard sheath because of insufficient rigidity, incurring undesired restriction in operation. The flexible cystoscope has some deficiencies, such as lacking of adequate work path, a relatively limited visual field, and inconvenient manipulation owing to friction between the flexible cystoscope and tissue.

In medical clinical diagnoses and treatment of renal disease, especially nephrolith, catheter nephroscope is necessary. A conventional catheter nephroscope apparatus comprises two types, rigid catheter nephroscope and flexible catheter nephroscope. The rigid catheter nephroscope has limited operation scope, for example, operates on one or two renal pelvises each time. Especially, treatment of complicated kidney stone is not efficacious, and rigid catheter nephroscope tends to hurt kidney and produce various complications. The flexible catheter nephroscope, also called catheter flexible nephroscope, has relatively small and unclear operational visual field without adequate operation room, and thus ultrasound lithotripsy and laser lithotripsy and so on can not be performed. The flexible catheter nephroscope has a long and thin body with low rigidity, subjecting to friction between tissues, and therefore gives rise to inflexible operation and cumbersome positioning and retention. It has to take much time to learn how to operate the flexible catheter nephroscope.

Furthermore, cystoscopes are necessary for medical clinical diagnoses and treatment of ureteral and bladder disease. A conventional ureteral cystoscope is classified as two types, rigid ureteral cystoscope and flexible ureteral cystoscope. The rigid ureteral cystoscope is the most widely employed, and typically has visual angles of 0 degree, 30 degrees, 70 degrees and 90 degrees. The visual angles are fixed and inadjustble, and visual field cuts are inevitable at least to some extent. The endoscopy with different visual angles needs to vary with different surgery operations, making operations more troublesome and increasing manufacture cost. The rigid ureteral cystoscope may hurt patients more seriously and produce comlications due to material. Moreover, the rigid ureteral cystoscope is an optical scopy, which has poor image quality of optical system, and thus continuous bladder wash is required to guarantee distinct surgery visual field. The fiber urethra cystoscope is a flexible cystoscope, and cannot enter a catheter or a bladder unless assistance of hard sheath because of insufficient rigidity, incurring undesired restriction in operation. The flexible urethra cystoscope has some deficiencies, such as lacking of adequate work path, a relatively limited visual field, and inconvenient manipulation.

### Summary of the Invention

An object of the utility model is to overcome deficiencies of the ureteroscope, and to provide an endoscope for diagnoses and treatment of ureteral disease, in particular to a rigid/flexible adjustable ureteroscope.

Another object of the utility model is to provide a rigid/flexible adjustable fibronephorscope.

A further object of the utility model is to provide an adjustable fiber urethra cystoscope.

The utility model combines a rigid ureteroscope and a flexible ureteroscope applied in the market or clinic to provide a rigid/flexible adjustable fiber ureteroscope for diagnoses and treatment of ureteral disease. The surgical operations are simplified, operation efficiency is raised, and manufacture cost is reduced. The ureteroscope is more economic and efficacious.

The utility model improves prior art fiber ureteroscope.

The utility model comprises a body, an operation shank and a rigid/flexible adjustment support rod.

The body 1 has a length of 50~150cm and an effective work diameter of 75~85 cm, and has a diameter of 5~18Fr, preferably 7~12Fr. The body may be flexible respectively at an upward angle of 180 degree, at a downward angle of 180 degree, and at a second downward angle of 130 degree for facilitating surgeries. The fiber ureteroscope body has a front part with an inclined surface. The inclined surface defines a hole with a diameter of 6~11Fr for ease of entering ureter and for avoiding hurt to tissues. The body includes a work channel, a water inlet/outlet channel, a fiber channel, a light source channel, and a rigid/flexible adjustment support rod channel. The rigid/flexible adjustment support rod channel is located in vicinity of a center of the fiber ureteroscope body.

The operation shank connects with the body. A work channel 2 is defined in a joint portion between the operation shank and the body. The work channel is super smooth and has a diameter of 3∼8Fr for appliance in laser fiber lithotripsy, ultrasound absorption lithotripsy and electrohydraulic lithotripsy, and endovenous apparatus operation such as biopsy forceps, pyelostomy and so on. A switch key 10 controls the work channel 2 to be opened or closed. A water inlet/outlet channel 3 is defined in the operation shank and opposite to the work channel 2. The water inlet/outlet channel 3 has a diameter of 3∼6Fr for ensuring adequate irrigation or assistance operation of other small endovenous apparatus. A switch key is provided on the water inlet/outlet channel 3 for controlling the water inlet/outlet channel 3. A control shaft 11 is formed between the work channel 2 and a light source interface 6. An operation control key 4 and a lock key 5 are respectively formed on opposite sides of the control shaft 11. The light source interface 6 is provided on a rear of the control shaft 11 and forms an angle of 90 degree with respect to the operation shank. An ocular 8 is provided on a rear of the light source interface 6 and forms an angle of 15∼75 degree, preferably 45 degree, with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body and the rigidity adjustment key 12. The rigid/flexible adjustment support rod body 14 is round and solid, and has a length of 50~150cm and an effective work length of 75~85cm, and has a diameter of 1~5Fr, preferably 3Fr. The rigid/flexible adjustment support rod is movable freely in the rigid/flexible adjustment support rod channel according to operation requirements, so that a front part of the endoscope becomes flexible to bend until the rigid/flexible adjustment support rod is drawn out completely for serving the rigid/flexible adjustment support rod channel as a work channel. An operator can recognize the flexible length of the fiber ureteroscope body according to a rod scale 15 on the rigid/flexible adjustment support rod.

According to another embodiment, a body 1' of a rigid/flexible adjustment fibronephorscope has a length of 20~50cm and a diameter of 12~18Fr; the rigid/flexible adjustment support rod 14' has a length of 20~50cm.

Further, the body 1' has a length of 35∼37cm and has a diameter of 16~18Fr. The body 1' may be flexible respectively at an upward angle of 180 degree, at a downward angle of 180 degree, and at a second downward angle of 130 degree for facilitating surgeries. The fiber ureteroscope body has a front part with an inclined surface. The inclined surface defines a hole with a diameter of 142~16Fr.

The rigid/flexible adjustment support rod body 14' is round and solid, and has a length of 20~50cm and has a diameter of 1~5Fr.

The rigid/flexible adjustment support rod body 14', preferably, has a length of 35~37cm and has a diameter of 3Fr.

According to another embodiment, a body 1" of a rigid/flexible adjustment fiber urethra cystoscope has a length of 20~100cm and a diameter of 9~21Fr. The body 1 " may be flexible respectively at an upward angle of 180 degree, at a downward angle of 180 degree, and at a second downward angle of 130 degree. The body 1 " has a front part with an inclined surface. The inclined surface defines a hole with a diameter of 16~18Fr. The rigid/flexible adjustment support rod body 14" is round and solid, and has a length of 20~100cm and has a diameter of 1~5Fr.

The body 1 " has a length of 42∼45cm and has a diameter of 18Fr.

The rigid/flexible adjustment support rod body 14", preferably, has a length of 42~45cm and has a diameter of 3Fr.

The utility model is specific for ureteral disease. Compared with the prior art, the utility model apparently overcomes defects of conventional ureteroscopes and raises surgical efficacy and economical merits with reduced cost. The utility model has following advantages: the rigidity adjustment key controls the rigid/flexible adjustment support rod to be movable in the fiber ureteroscope body, so that the fiber ureteroscope body can become rigid to provide adequate rigidity to overcome resistance, avoiding needs of hard sheath during extension into a ureter. In diagnoses and treatment operation, movement of the rigid/flexible adjustment support rod makes a front part of the endoscope becomes flexible, and the flexible front part is variable in length for accommodating different cases. In order to meet different requirements, the rigid/flexible adjustment support rod can freely pass through the rigid/flexible adjustment support rod channel, and can be drawn out completely in such a way that the rigid/flexible adjustment support rod channel may serve as a work channel. The operator can recognize the length which converts from rigid to flexible or from flexible to rigid with a scale in the rigid/flexible adjustment support rod. The endoscope may become flexible to bend at a large angle, overcoming the defect of visual field cuts due to angle limitation. An outer diameter of fiber ureteroscope body may increase apparently without restriction of the outer sheath, and the number and size of work channels increase, which is combined with gripping tongs, biopsy forceps, basket manipulation and laser fiber for benefiting for various medical surgery and ensuring a large and clear enough surgical visual field, thereby simplifying fabrication of a flexible endoscope and avoiding hurt to tissues. The endoscope combines rigid endoscope and fiber flexible endoscope, so as to reduce apparatus acquisition cost and to promote medical diagnoses and treatment capability, achieving desired utility and market value.

The utility model provides a rigid/flexible adjustable fibronephorscope by virtue of scale adjustment of a support rod relative to a body. Compared with the prior art, the utility model overcomes defects of various nephroscopes, raises surgical efficacy and economic merits with reduced cost. Hard sheath is not needed while entering a kidney. In operation, movement of the rigid/flexible adjustment support rod makes a front part of the endoscope becomes flexible, and the flexible front part is variable in length for accommodating different cases. An outer diameter of the fibronephorscope body may increase apparently, and the number and size of work channels increase. The endoscope combines advantages of rigid fibronephorscope and flexible fibronephorscope.

The utility model provides a rigid/flexible adjustable fiber urethra cystoscope for diagnoses and treatment of ureter and bladder disease by virtue of scale adjustment of a support rod relative to a body. Hard sheath is not needed while entering a ureter or a bladder. In operation, movement of the rigid/flexible adjustment support rod makes a front part of the endoscope becomes flexible, and an operator can recognize the length of the flexible front part by a rod scale on the support rod. The endoscope combines advantages of rigid fibronephorscope and flexible fibronephorscope. Compared with the prior art, the utility model overcomes defects of various urethra cystoscopes, raises surgical efficacy and economic merits with reduced cost.

### Brief Description of the Drawings

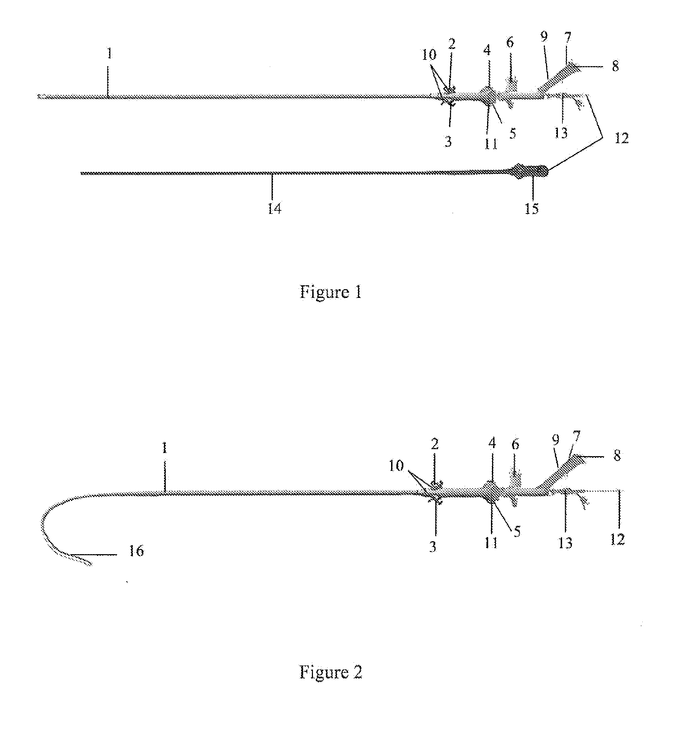

Figure 1 is a schematic diagram of a rigid/flexible adjustable ureteroscope,

wherein 1 denotes body, 2 denotes work channel, 3 denotes a water inlet/outlet channel, 4 denotes operation control key, 5 denotes lock key, 6 denotes light source interface, 7 denotes focus button, 8 denotes ocular, 9 denotes focus scale, 10 denotes switch key, 11 denotes control shaft, 12 denotes rigidity adjustment key, 13 denotes support rod switch key, 14 denotes support rod, 15 denotes scale;

Figure 2 schematically shows movement of the rigid/flexible adjustment support rod under control of rigidity adjustment key making a front part of the endoscope becomes from rigid to flexible,

wherein 1 denotes body, 2 denotes work channel, 3 denotes a water inlet/outlet channel, 4 denotes operation control key, 5 denotes lock key, 6 denotes light source interface, 7 denotes focus button, 8 denotes ocular, 9 denotes focus scale, 10 denotes switch key, 11 denotes control shaft, 12 denotes rigidity adjustment key, 13 denotes support rod switch key, 16 denotes flexible front part;

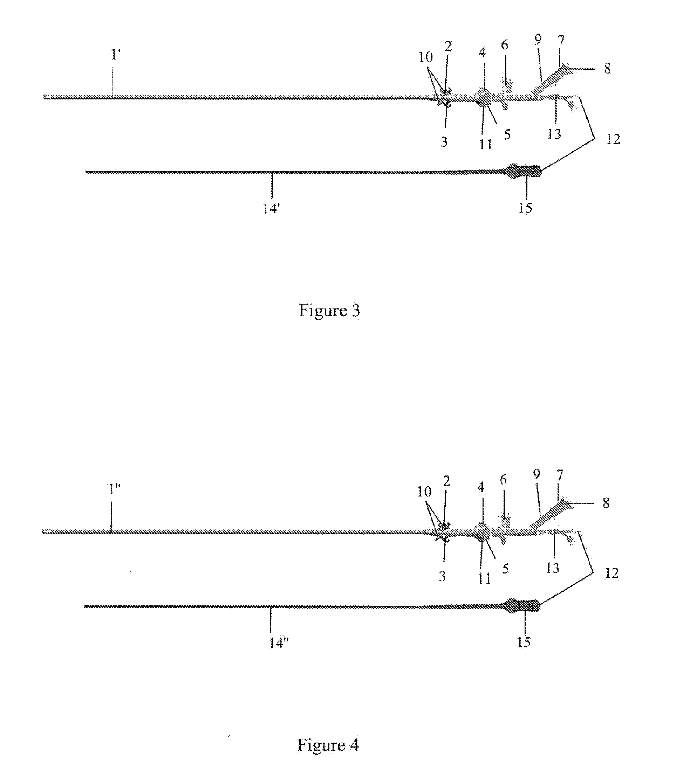

Figure 3 is a schematic diagram of a rigid/flexible adjustable fibronephorscope,

wherein 1' denotes body, 14' denotes support rod, and other reference numerals are identical to those in Figures 1 and 2;

Figure 4 is a schematic diagram of a rigid/flexible adjustable fiber urethra cystoscope,

wherein 1 " denotes body, 14" denotes support rod, and other reference numerals are identical to those in Figures 1 and 2.

### Detailed Description of the Invention

The utility model is further described in combination with the drawings.

Embodiment 1

Referring to Figures 1 and 2, the utility model improves flexible ureteroscopes and rigid ureteroscopes to make a rigid/flexible adjustable fiber ureteroscope for diagnoses and treatment of ureteral disease. The rigid/flexible adjustable fiber ureteroscope comprises a fiber ureteroscope body, an operation shank and a rigid/flexible adjustment support rod.

The fiber ureteroscope body 1 has a length of 50cm and a diameter of 5Fr, and may be flexible respectively at an upward angle of 180 degree, at a downward angle of 180 degree, and at a second downward angle of 130 degree. The fiber ureteroscope body has a front part with an inclined surface. The inclined surface defines a hole with a diameter of 6Fr. The fiber ureteroscope body includes a work channel, a water inlet/outlet channel, a fiber channel, a light source channel, and a rigid/flexible adjustment support rod channel. The rigid/flexible adjustment support rod channel is located in vicinity of a center of the fiber ureteroscope body.

The operation shank connects with the fiber ureteroscope body. A work channel 2 is defined in a joint portion between the operation shank and the fiber ureteroscope body. The work channel is super smooth and has a diameter of 3Fr. The switch key 10 controls the work channel 2 to be opened or closed. A water inlet/outlet channel 3 is defined in the operation shank and opposite to the work channel 2. The water inlet/outlet channel 3 has a diameter of 3Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 15 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14 and the rigidity adjustment key 12. The rigid/flexible adjustment support rod body 14 is round and solid, and has an effective work length of 50cm and a diameter of 1Fr. The rigid/flexible adjustment support rod is movable freely in the rigid/flexible adjustment support rod channel according to operation requirements, so that a front part 16 of the endoscope becomes flexible to bend until the rigid/flexible adjustment support rod is drawn out completely for serving the rigid/flexible adjustment support rod channel as a work channel. An operator can recognize the flexible length of the fiber ureteroscope body according to a rod scale 15 on the rigid/flexible adjustment support rod.

The utility model is specific for ureteral disease, and has following advantages: the rigidity adjustment key controls the rigid/flexible adjustment support rod to be movable in the fiber ureteroscope body, so that the fiber ureteroscope body can become rigid to provide adequate rigidity to overcome resistance, avoiding needs of hard sheath during extension into a ureter; in operation, movement of the rigid/flexible adjustment support rod makes a front part of the endoscope becomes flexible, and the flexible front part is variable in length for accommodating different cases. In order to meet different requirements, the rigid/flexible adjustment support rod can freely pass through the rigid/flexible adjustment support rod channel, and can be drawn out completely in such a way that the rigid/flexible adjustment support rod channel may serve as a work channel. The operator can recognize the length which converts from rigid to flexible or from flexible to rigid with a scale in the rigid/flexible adjustment support rod. The endoscope may become flexible to bend at a large angle, overcoming the defect of visual field cuts due to angle limitation. An outer diameter of fiber ureteroscope body may increase apparently without restriction of the outer sheath, and the number and size of work channels increase, which is combined with gripping tongs, biopsy forceps, basket manipulation and laser fiber for benefiting for various medical surgery and ensuring a large and clear enough surgical visual field, thereby simplifying fabrication of a flexible endoscope and avoiding hurt to tissues.

Embodiment 2

Referring to Figures 1 and 2, the utility model improves flexible ureteroscopes and rigid ureteroscopes to make a rigid/flexible adjustable fiber ureteroscope for diagnoses and treatment of ureteral disease. The rigid/flexible adjustable fiber ureteroscope comprises a fiber ureteroscope body, an operation shank and a rigid/flexible adjustment support rod.

The fiber ureteroscope body 1 has a length of 150cm and a diameter of 18Fr, and may be flexible respectively at an upward angle of 180 degree, at a downward angle of 180 degree, and at a second downward angle of 130 degree. The fiber ureteroscope body has a front part with an inclined surface. The inclined surface defines a hole with a diameter of 11Fr. The fiber ureteroscope body includes a work channel, a water inlet/outlet channel, a fiber channel, a light source channel, and a rigid/flexible adjustment support rod channel. The rigid/flexible adjustment support rod channel is located in vicinity of a center of the fiber ureteroscope body.

The operation shank connects with the fiber ureteroscope body. A work channel 2 is defined in a joint portion between the operation shank and the fiber ureteroscope body. The work channel is super smooth and has a diameter of 8Fr. The switch key 10 controls the work channel 2 to be opened or closed. A water inlet/outlet channel 3 is defined in the operation shank and opposite to the work channel 2. The water inlet/outlet channel 3 has a diameter of 6Fr. A switch key is provided on the water inlet/outlet channel 3 for controlling the water inlet/outlet channel 3. A control shaft 11 I is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 75 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14 and the rigidity adjustment key 12. The rigid/flexible adjustment support rod body 14 is round and solid, and has an effective work length of 150cm and a diameter of 5Fr. The rigid/flexible adjustment support rod is movable freely in the rigid/flexible adjustment support rod channel according to operation requirements, so that a front part 16 of the endoscope becomes flexible to bend until the rigid/flexible adjustment support rod is drawn out completely for serving the rigid/flexible adjustment support rod channel as a work channel. An operator can recognize the flexible length of the fiber ureteroscope body according to a rod scale 15 on the rigid/flexible adjustment support rod.

Compared with the prior art, the utility model apparently overcomes defects of conventional ureteroscope and raises surgical efficacy and economical merits with reduced cost.

Embodiment 3

The rigid/flexible adjustable fiber ureteroscope of the utility model comprises a fiber ureteroscope body, an operation shank and a rigid/flexible adjustment support rod.

The fiber ureteroscope body 1 has a length of 85cm and a diameter of 12Fr, and may be flexible respectively at an upward angle of 180 degree, at a downward angle of 180 degree, and at a second downward angle of 130 degree for facilitating surgeries. The fiber ureteroscope body has a front part with an inclined surface. The inclined surface defines a hole with a diameter of 11Fr for ease of entering a ureter without hurting tissues. The fiber ureteroscope body includes a work channel, a water inlet/outlet channel, a fiber channel, a light source channel, and a rigid/flexible adjustment support rod channel. The rigid/flexible adjustment support rod channel is located in vicinity of a center of the fiber ureteroscope body.

The operation shank connects with the fiber ureteroscope body. A work channel 2 is defined in a joint portion between the operation shank and the fiber ureteroscope body. The work channel is super smooth and has a diameter of 8Fr for appliance in laser fiber lithotripsy, ultrasound absorption lithotripsy and electrohydraulic lithotripsy, and endovenous apparatus operation such as biopsy forceps, pyelostomy and so on. The switch key 10 controls the work channel 2 to be opened or closed. A water inlet/outlet channel 3 is defined in the operation shank and opposite to the work channel 2. The water inlet/outlet channel 3 has a diameter of 6Fr for ensuring adequate irrigation or assistance operation of other small endovenous apparatus. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 45 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14 and the rigidity adjustment key 12. The rigid/flexible adjustment support rod body 14 is round and solid, and has a length of 85cm and a diameter of 3Fr. The rigid/flexible adjustment support rod is movable freely in the rigid/flexible adjustment support rod channel under control of the support rod switch key according to operation requirements, so that a front part 16 of the endoscope becomes flexible to bend until the rigid/flexible adjustment support rod is drawn out completely for serving the rigid/flexible adjustment support rod channel as a work channel. An operator can recognize the flexible length of the fiber ureteroscope body according to a rod scale 15 on the rigid/flexible adjustment support rod.

The utility model is specific for ureteral disease. Compared with the prior art, the utility model apparently overcomes defects of conventional ureteroscope, raises surgical efficacy and capability of diagnosis and treat, avoids to hurt tissues, and reduces apparatus acquisition cost.

Embodiment 4

Referring to Figure 3, the utility model improves flexible catheter nephroscopes and rigid catheter nephroscopes to make a rigid/flexible adjustable fibronephorscope. The rigid/flexible adjustable fibronephorscope comprises a fibronephorscope body, an operation shank and a rigid/flexible adjustment support rod.

The fibronephorscope body 1' has a length of 20cm and a diameter of 12Fr, and may be flexible respectively at an upward angle of 180 degree, at a downward angle of 180 degree, and at a second downward angle of 130 degree for facilitating surgeries. The fibronephorscope body has a front part with an inclined surface. The inclined surface defines a hole with a diameter of 14Fr. The fiber ureteroscope body includes a work channel, a water inlet/outlet channel, a fiber channel, a light source channel, and a rigid/flexible adjustment support rod channel. The rigid/flexible adjustment support rod channel is located in vicinity of a center of the fibronephorscope body.

The operation shank connects with the fibronephorscope body 1'. A work channel 2 is defined in a joint portion between the operation shank and the fibronephorscope body 1'. The work channel is super smooth and has a diameter of 3Fr. A switch key 10 controls the work channel 2 to be opened or closed. A water inlet/outlet channel 3 is defined in the operation shank and opposite to the work channel 2. The water inlet/outlet channel 3 has a diameter of 3Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 15 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14' and the rigidity adjustment key 12. The rigid/flexible adjustment support rod body 14' is round and solid, and has a length of 20cm and a diameter of 1Fr. The rigid/flexible adjustment support rod is movable freely in the rigid/flexible adjustment support rod channel under control of the support rod switch key according to operation requirements, so that a front part 16 of the endoscope becomes flexible to bend until the rigid/flexible adjustment support rod is drawn out completely for serving the rigid/flexible adjustment support rod channel as a work channel. An operator can recognize the flexible length of the fibronephorscope body according to a rod scale 15 on the rigid/flexible adjustment support rod.

The utility model can overcome defects of prior art nephroscope apparently, for example, the rigidity adjustment key controls the rigid/flexible adjustment support rod to be movable in the fibronephorscope body, so that the fibronephorscope body can become rigid to provide adequate rigidity to overcome resistance, avoiding needs of hard sheath during operation. Movement of the rigid/flexible adjustment support rod makes a front part of the endoscope becomes flexible, and the flexible front part is variable in length for accommodating different cases. In order to meet different requirements, the rigid/flexible adjustment support rod can freely pass through the rigid/flexible adjustment support rod channel, and can be drawn out completely in such a way that the rigid/flexible adjustment support rod channel may serve as a work channel. The operator can recognize the length which converts from rigid to flexible or from flexible to rigid with a rod scale in the rigid/flexible adjustment support rod. The front part of the endoscope may become flexible to bend at a large angle, overcoming the defect of visual field cuts due to angle limitation. An outer diameter of the fibronephorscope body may increase apparently, and the number and size of work channels increase, which is combined with gripping tongs, biopsy forceps, basket manipulation, laser fiber and so on for benefiting for various medical surgery and ensuring a large and clear enough surgical visual field, thereby simplifying fabrication of a flexible endoscope and avoiding hurt to tissues.

Embodiment 5

The utility model improves flexible catheter nephroscopes and rigid catheter nephroscopes to make a rigid/flexible adjustable fibronephorscope. The rigid/flexible adjustable fibronephorscope comprises a fibronephorscope body 1', an operation shank and a rigid/flexible adjustment support rod 14'.

The fibronephorscope body 1' has a length of 50cm and a diameter of 18Fr, and may be flexible respectively at an upward angle of 180 degree, at a downward angle of 180 degree, and at a second downward angle of 130 degree for facilitating surgeries. The fibronephorscope body has a front part with an inclined surface. The inclined surface defines a hole with a diameter of 16Fr for ease of entering renal fistula and for avoiding hurt to tissues. The fibronephorscope body includes a work channel, a water inlet/outlet channel, a fiber channel, a light source channel, and a rigid/flexible adjustment support rod channel. The rigid/flexible adjustment support rod channel is located in vicinity of a center of the fibronephorscope body.

The operation shank connects with the fibronephorscope body 1'. A work channel 2 is defined in a joint portion between the operation shank and the fibronephorscope body. The work channel is super smooth and has a diameter of 8Fr. A switch key 10 controls the work channel 2 to be opened or closed. A water inlet/outlet channel 3 is defined in the operation shank and opposite to the work channel 2. The water inlet/outlet channel 3 has a diameter of 6Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 75 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod 14' to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14' and the rigidity adjustment key 12. The rigid/flexible adjustment support rod body 14' is round and solid, and has a length of 50cm and a diameter of 5Fr. The rigid/flexible adjustment support rod is movable freely in the rigid/flexible adjustment support rod channel according to operation requirements, so that a front part 16 of the endoscope becomes flexible to bend until the rigid/flexible adjustment support rod is drawn out completely for serving the rigid/flexible adjustment support rod channel as a work channel. An operator can recognize the flexible length of the fibronephorscope body according to a rod scale 15 on the rigid/flexible adjustment support rod.

The utility model is specific for renal disease. Compared with the prior art, the utility model apparently overcomes defects of conventional nephroscope and raises surgical efficacy and economical merits with reduced cost.

Embodiment 6

The utility model improves flexible catheter nephroscopes and rigid catheter nephroscopes to make a rigid/flexible adjustable fibronephorscope. The rigid/flexible adjustable fibronephorscope comprises a fibronephorscope body, an operation shank and a rigid/flexible adjustment support rod.

The fibronephorscope body 1' has a length of 35cm and a diameter of 16Fr, and may be flexible respectively at an upward angle of 180 degree, at a downward angle of 180 degree, and at a second downward angle of 130 degree for facilitating surgeries. The fibronephorscope body has a front part with an inclined surface. The inclined surface defines a hole with a diameter of 16Fr for ease of entering renal fistula and for avoiding hurt of tissues. The fibronephorscope body includes a work channel, a water inlet/outlet channel, a fiber channel, a light source channel, and a rigid/flexible adjustment support rod channel. The rigid/flexible adjustment support rod channel is located in vicinity of a center of the fibronephorscope body.

The operation shank connects with the fibronephorscope body 1'. A work channel 2 is defined in a joint portion between the operation shank and the fibronephorscope body. The work channel is super smooth and has a diameter of 8Fr. A switch key 10 controls the work channel 2 to be opened or closed. A water inlet/outlet channel 3 is defined in the operation shank and opposite to the work channel 2. The water inlet/outlet channel 3 has a diameter of 6Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 75 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod 14' to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14' and the rigidity adjustment key 12. The rigid/flexible adjustment support rod body 14' is round and solid, and has a length of 35cm and a diameter of 3Fr. The rigid/flexible adjustment support rod is movable freely in the rigid/flexible adjustment support rod channel according to operation requirements, so that a front part 16 of the endoscope becomes flexible to bend until the rigid/flexible adjustment support rod is drawn out completely for serving the rigid/flexible adjustment support rod channel as a work channel. An operator can recognize the flexible length of the fibronephorscope body according to a rod scale 15 on the rigid/flexible adjustment support rod.

Compared with the prior art, the utility model apparently overcomes defects of conventional nephroscope and raises surgical efficacy and economical merits with reduced cost.

Embodiment 7

Referring to Figure 4, the utility model improves conventional flexible urethra cystoscopes and rigid urethra cystoscopes to make a rigid/flexible adjustable urethra cystoscope. The rigid/flexible adjustable urethra cystoscope comprises a urethra cystoscope body, an operation shank and a rigid/flexible adjustment support rod.

The urethra cystoscope body 1 " has a length of 20cm and a diameter of 9Fr, and may be flexible respectively at an upward angle of 180 degree, at a downward angle of 180 degree, and at a second downward angle of 130 degree. The urethra cystoscope body has a front part with an inclined surface. The inclined surface defines a hole with a diameter of 16Fr. The urethra cystoscope body includes a work channel, a water inlet/outlet channel, a fiber channel, a light source channel, and a rigid/flexible adjustment support rod channel. The rigid/flexible adjustment support rod channel is located in vicinity of a center of the urethra cystoscope body.

The operation shank connects with the urethra cystoscope body. A work channel 2 is defined in a joint portion between the operation shank and the urethra cystoscope body. The work channel is super smooth and has a diameter of 3Fr. The switch key 10 controls the work channel 2 to be opened or closed. A water inlet/outlet channel 3 is defined in the operation shank and opposite to the work channel 2. The water inlet/outlet channel 3 has a diameter of 3Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 15 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14", a rod scale and the rigidity adjustment key 12. The rigid/flexible adjustment support rod body 14" is round and solid, and has a length of 20cm and a diameter of 1Fr. The rigid/flexible adjustment support rod is movable freely in the rigid/flexible adjustment support rod channel according to operation requirements, so that a front part of the endoscope becomes flexible to bend until the rigid/flexible adjustment support rod is drawn out completely for serving the rigid/flexible adjustment support rod channel as a work channel. An operator can recognize the flexible length of the urethra cystoscope body according to the rod scale 15 on the rigid/flexible adjustment support rod.

The utility model has the rigidity adjustment key for controlling the rigid/flexible adjustment support rod to be movable in the urethra cystoscope body, so that the urethra cystoscope body can become rigid to provide adequate rigidity to overcome resistance, avoiding needs of hard sheath during extension into a ureter or a bladder; in operation, movement of the rigid/flexible adjustment support rod makes a front part of the endoscope becomes flexible, and the flexible front part is variable in length for accomplishing required operations. In order to meet different requirements, the rigid/flexible adjustment support rod can freely pass through the rigid/flexible adjustment support rod channel, and can be drawn out completely in such a way that the rigid/flexible adjustment support rod channel may serve as a work channel. The operator can recognize the length which converts from rigid to flexible or from flexible to rigid with the rod scale in the rigid/flexible adjustment support rod. The endoscope may become flexible to bend at a large angle, overcoming the defect of visual field cuts due to angle limitation. An outer diameter of urethra cystoscope body may increase apparently without restriction of the outer sheath, and the number and size of work channels increase, which is combined with gripping tongs, biopsy forceps, basket manipulation and laser fiber for benefiting for various medical surgeries and ensuring a large and clear enough surgical visual field, thereby simplifying fabrication of a flexible endoscope and avoiding hurt to tissues. Compared with the prior art, the utility model apparently overcomes defects of conventional urethra cystoscope and raises surgical efficacy and economical merits with reduced cost.

Embodiment 8

The utility model improves conventional flexible urethra cystoscopes and rigid urethra cystoscopes to make a rigid/flexible adjustable urethra cystoscope. The rigid/flexible adjustable urethra cystoscope comprises a urethra cystoscope body, an operation shank and a rigid/flexible adjustment support rod.

The urethra cystoscope body 1" has a length of 100cm and a diameter of 21Fr, and may be flexible respectively at an upward angle of 180 degree, at a downward angle of 180 degree, and at a second downward angle of 130 degree. The urethra cystoscope body has a front part with an inclined surface. The inclined surface defines a hole with a diameter of 18Fr. The urethra cystoscope body includes a work channel, a water inlet/outlet channel, a fiber channel, a light source channel, and a rigid/flexible adjustment support rod channel. The rigid/flexible adjustment support rod channel is located in vicinity of a center of the urethra cystoscope body.

The operation shank connects with the urethra cystoscope body. A work channel 2 is defined in a joint portion between the operation shank and the urethra cystoscope body. The work channel is super smooth and has a diameter of 8Fr. A switch key 10 controls the work channel 2 to be opened or closed. A water inlet/outlet channel 3 is defined in the operation shank and opposite to the work channel 2. The water inlet/outlet channel 3 has a diameter of 6Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 75 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14", a rod scale and the rigidity adjustment key 12. The rigid/flexible adjustment support rod body 14" is round and solid, and has a length of 100cm and a diameter of 5Fr. The rigid/flexible adjustment support rod is movable freely in the rigid/flexible adjustment support rod channel according to operation requirements, so that a front part of the endoscope becomes flexible to bend until the rigid/flexible adjustment support rod is drawn out completely for serving the rigid/flexible adjustment support rod channel as a work channel. An operator can recognize the flexible length of the urethra cystoscope body according to the rod scale 15 on the rigid/flexible adjustment support rod.

The utility model has the rigidity adjustment key for controlling the rigid/flexible adjustment support rod to be movable in the urethra cystoscope body, so that the urethra cystoscope body can become rigid to provide adequate rigidity to overcome resistance, avoiding needs of hard sheath during extension into a ureter or a bladder; in operation, movement of the rigid/flexible adjustment support rod makes a front part of the endoscope becomes flexible, and the flexible front part is variable in length for accomplishing required operations. In order to meet different requirements, the rigid/flexible adjustment support rod can freely pass through the rigid/flexible adjustment support rod channel, and can be drawn out completely in such a way that the rigid/flexible adjustment support rod channel may serve as a work channel. The operator can recognize the length which converts from rigid to flexible or from flexible to rigid with the rod scale in the rigid/flexible adjustment support rod. The endoscope may become flexible to bend at a large angle, overcoming the defect of visual field cuts due to angle limitation. An outer diameter of urethra cystoscope body may increase apparently without restriction of the outer sheath, and the number and size of work channels increase, benefiting for various medical surgeries and ensuring a large and clear enough surgical visual field, thereby avoiding hurt to tissues.

Embodiment 9

The utility model improves conventional flexible urethra cystoscopes and rigid urethra cystoscopes to make a rigid/flexible adjustable urethra cystoscope. The rigid/flexible adjustable urethra cystoscope comprises a urethra cystoscope body, an operation shank and a rigid/flexible adjustment support rod.

The urethra cystoscope body 1" has a length of 42-45cm and a diameter of 18Fr, and may be flexible respectively at an upward angle of 180 degree, at a downward angle of 180 degree, and at a second downward angle of 130 degree. The urethra cystoscope body has a front part with an inclined surface. The inclined surface defines a hole with a diameter of 18Fr. The urethra cystoscope body includes a work channel, a water inlet/outlet channel, a fiber channel, a light source channel, and a rigid/flexible adjustment support rod channel. The rigid/flexible adjustment support rod channel is located in vicinity of a center of the urethra cystoscope body.

The operation shank connects with the urethra cystoscope body. A work channel 2 is defined in a joint portion between the operation shank and the urethra cystoscope body. The work channel is super smooth and has a diameter of 8Fr. A switch key 10 controls the work channel 2 to be opened or closed. A water inlet/outlet channel 3 is defined in the operation shank and opposite to the work channel 2. The water inlet/outlet channel 3 has a diameter of 6Fr. A switch key is provided on the water inlet/outlet channel 3 for controlling the water inlet/outlet channel 3. A control shaft 11 is formed between the work channel 2 and a light source interface 6. An operation control key 4 is formed on one side of the control shaft 11, and a lock key 5 is formed on another side opposite to the operation control key 4. The light source interface 6 is provided on a rear of the control shaft and forms an angle of 90 degree with respect to the operation shank. An ocular 8 is provided on a rear of the light source interface 6 and forms an angle of 45 degree with respect to the operation shank. A focus button 7 is provided between the light source interface 6 and the ocular 8. A focus scale 9 is formed on the focus button 7. A rigidity adjustment key 12 is provided on a rear end of the operation shank for allowing the rigid/flexible adjustment support rod to be movable freely in the rigid/flexible adjustment support rod channel. A support rod switch key 13 is provided on a distal end of the operation shank for controlling and stabilizing the rigid/flexible adjustment support rod, and preventing reflux of irrigating solutions during operation.

The rigid/flexible adjustment support rod comprises a rigid/flexible adjustment support rod body 14", a rod scale and the rigidity adjustment key. The rigid/flexible adjustment support rod body 14" is round and solid, and has a length of 42-45cm and a diameter of 3Fr. The rigid/flexible adjustment support rod is movable freely in the rigid/flexible adjustment support rod channel according to operation requirements, so that a front part of the endoscope becomes flexible to bend until the rigid/flexible adjustment support rod is drawn out completely for serving the rigid/flexible adjustment support rod channel as a work channel. An operator can recognize the flexible length of the urethra cystoscope body according to the rod scale 15 on the rigid/flexible adjustment support rod.

The utility model has the rigidity adjustment key for controlling the rigid/flexible adjustment support rod to be movable in the urethra cystoscope body, so that the urethra cystoscope body can become rigid to provide adequate rigidity to overcome resistance, avoiding needs of hard sheath during extension into a ureter or a bladder; in operation, movement of the rigid/flexible adjustment support rod makes a front part of the endoscope becomes flexible, and the flexible front part is variable in length for accomplishing required operations. In order to meet different requirements, the rigid/flexible adjustment support rod can freely pass through the rigid/flexible adjustment support rod channel, and can be drawn out completely in such a way that the rigid/flexible adjustment support rod channel may serve as a work channel. The operator can recognize the length which converts from rigid to flexible or from flexible to rigid with the rod scale in the rigid/flexible adjustment support rod. The endoscope may become flexible to bend at a large angle, overcoming the defect of visual field cuts due to angle limitation. An outer diameter of urethra cystoscope body may increase apparently without restriction of the outer sheath, and the number and size of work channels increase, benefiting for various medical surgeries and ensuring a large and clear enough surgical visual field, thereby avoiding hurt to tissues.

## Claims

1. A rigid/flexible adjustable ureteroscope comprising a body, an operation shank and a rigid/flexible adjustment support rod, and **characterized in that**, the body (1) includes a work channel, a water inlet/outlet channel, a fiber channel, a light source channel, and a rigid/flexible adjustment support rod channel which is located in vicinity of a center of the body,
wherein the operation shank connects with the body (1), a work channel (2) is defined in a joint portion between the operation shank and the body, the switch key (10) is provided to opened or close the work channel (2), the water inlet/outlet channel (3) is defined in the operation shank and opposite to the work channel (2), a control shaft (11) is provided between the work channel and a light source interface (6), an operation control key (4) and a lock key (5) are respectively formed on opposite sides of the control shaft (11), the light source interface (6) is provided on a rear of the control shaft and forming an angle of 90 degree with respect to the operation shank, an ocular (8) is provided on a rear of the light source interface (6) and forms an angle ranged of 15∼75 degree with respect to the operation shank, a focus button (7) is provided between the light source interface (6) and the ocular (8), a focus scale (9) is formed on the focus button, a rigidity adjustment key (12) is provided on a rear end of the operation shank, a support rod switch key (13) is provided on a distal end of the operation shank; and
wherein the rigidity adjustment support rod comprises a rigid/flexible adjustment support rod body (14) and the rigidity adjustment key (12), and a rod scale (15) is provided on the support rod.

2. The rigid/flexible adjustable ureteroscope according to claim 1, **characterized in that** the body (1) has a length of 50~150cm and a diameter of 5~18Fr, and the rigid/flexible adjustment support rod has a length of 50~150cm.

3. The rigid/flexible adjustable ureteroscope according to claim 2, **characterized in that** the rigid/flexible adjustment support rod body (14) has a length of 75∼85cm and a diameter of 7~12Fr, and the body is flexible respectively at an upward angle of 180 degree, at a downward angle of 180 degree, and at a second downward angle of 130 degree, and the body has a front part with an inclined surface, the inclined surface defining a hole with a diameter of 6~11Fr.

4. The rigid/flexible adjustable ureteroscope according to claim 2, **characterized in that** the work channel is super smooth and has a diameter of 3∼8Fr, and the water inlet/outlet channel has a diameter of 3~6Fr.

5. The rigid/flexible adjustable ureteroscope according to claim 1, **characterized in that** the ocular forms an angle of 45 degree with respect to the operation shank.

6. The rigid/flexible adjustable ureteroscope according to claim 1, **characterized in that** the rigid/flexible adjustment support rod body is round and solid, and has a length of 50~150cm and a diameter of 1~5Fr.

7. The rigid/flexible adjustable ureteroscope according to claim 2, **characterized in that** the rigid/flexible adjustment support rod body has a length of 75~85cm and a diameter of 3Fr.

8. The rigid/flexible adjustable ureteroscope according to claim 1, **characterized in that** the rigid/flexible adjustment support rod body is movable in and is able to be drawn out from the rigid/flexible adjustment support rod channel for serving the rigid/flexible adjustment support rod channel as the work channel.

9. The rigid/flexible adjustable ureteroscope according to claim 1, **characterized in that** the body comprises a rigid/flexible adjustable fibronephorscope body (1') which has a length of 20~50cm and a diameter of 12~18Fr; and the rigid/flexible adjustment support rod body (14') has a length of 20~50cm.

10. The rigid/flexible adjustable ureteroscope according to claim 9, **characterized in that** the rigid/flexible adjustable fibronephorscope body (1') has a length of 35~37cm and a diameter of 16~18Fr, and the body is flexible respectively at an upward angle of 180 degree, at a downward angle of 180 degree, and at a second downward angle of 130 degree, and the body has a front part with an inclined surface, the inclined surface defining a hole with a diameter of 14~16Fr.

11. The rigid/flexible adjustable ureteroscope according to claim 9, **characterized in that** the rigid/flexible adjustment support rod body (14') is round and solid, and has a length of 20~50cm and a diameter of 1~5Fr.

12. The rigid/flexible adjustable ureteroscope according to claim 11, **characterized in that** the rigid/flexible adjustment support rod body (14') has a length of 35~37cm and a diameter of 3Fr.

13. The rigid/flexible adjustable ureteroscope according to claim 1, **characterized in that** the body comprises a rigid/flexible adjustment fiber urethra cystoscope body (1") which has a length of 20~100cm and a diameter of 9~21Fr, the body is flexible respectively at an upward angle of 180 degree, at a downward angle of 180 degree, and at a second downward angle of 130 degree, the body has a front part with an inclined surface, the inclined surface defining a hole with a diameter of 14~16Fr, and the rigid/flexible adjustment support rod body (14') is round and solid and has a length of 20~100cm and a diameter of 1~5Fr.

14. The rigid/flexible adjustable ureteroscope according to claim 13, **characterized in that** the body (1") has a length of 42~45cm and a diameter of 18Fr.

15. The rigid/flexible adjustable ureteroscope according to claim 15, **characterized in that** the body (14") has a length of 42∼45cm and a diameter of 3Fr.
